# EUROPÄISCHE PATENTSCHRIFT

(11) **EP 4 116 988 B1**
(45) Veröffentlichungstag und Bekanntmachung des Hinweises auf die Patenterteilung: **18.03.2026**
(21) Anmeldenummer: 21183760.4
(22) Anmeldetag: 05.07.2021
(51) Int. Cl.: G16H 20/17, G16H 40/63, G16H 50/20

(54) **SYSTEM ZUR ÜBERWACHUNG DER EINNAHME VON SUBSTANZEN**
SYSTEM FOR MONITORING THE INTAKE OF SUBSTANCES
SYSTÈME DE SURVEILLANCE DE L'ABSORPTION DE SUBSTANCES

(43) Veröffentlichungstag der Anmeldung: 11.01.2023
(73) Patentinhaber: Aptar Radolfzell GmbH, 78315 Radolfzell (DE)
(72) Erfinder: Körner, Joachim, 88690 Uhldingen-Mühlhofen (DE); Storz-Sontheimer, Julien, 78244 Gottmadingen (DE)
(74) Vertreter: Witte, Weller und Partner Patentanwälte mbB Stuttgart

(56) Entgegenhaltungen:
- US-A1- 2014 346 186
- US-A1- 2015 061 867
- US-A1- 2019 387 796
- US-A1- 2021 134 421

## Beschreibung

Die Erfindung betrifft ein System zur Überwachung der Einnahme von Substanzen, insbesondere von pharmazeutischen Substanzen oder von Nikotin.

Zur Einbringung aktiver Substanzen in den Körper sind eine Vielzahl von Wegen üblich, so insbesondere die Einbringung auf oralem oder nasalem Wege in Form einer Flüssigkeit, beispielsweise in Tropfenform oder in Form eines Sprühstrahl, aber auch in Form von Tabletten oder Kaugummis, die ihren Wirkstoff nach oraler Aufnahme im Mundraum oder im Magen des Benutzers freigeben.

Insbesondere im Bereich der Einbringung von Nikotin in den Körper ist es darüber hinaus üblich, hierfür Pflaster zu verwenden, die auf der Haut des Nutzers angebracht werden und die Substanz über einen längeren Zeitraum dem Körper des Nutzers zuführen.

Bei vielen Substanzen und insbesondere bei Nikotin ist es gewünscht, die Menge der bereits in den menschlichen Körper eingebrachten Substanz abschätzen zu können, beispielsweise um einen vorgegebenen Zielwert der eingebrachten Substanz in einem definierten Zeitraum zu erreichen oder um einen vorgegebenen Maximalwert nicht zu überschreiten.

So ist es insbesondere im Kontext von Nikotin wünschenswert, dass der Nutzer zum Zwecke der Begrenzung des eigenen Konsums oder zum Zwecke der Entwöhnung einen Überblick darüber bewahrt, wie viel Nikotin er oder sie in einem definierten Zeitraum, wie beispielsweise am aktuellen Tag, bereits zu sich genommen haben.

Aus dem Stand der Technik sind bereits Spender bekannt, beispielsweise Flüssigkeitsspender, die mit elektronischen Mitteln die Nutzung des Spenders erfassen, so dass die Nutzung des Spenders und die eingenommene Flüssigkeitsmenge am Spender oder einem hiermit verbundenen Smartphone abgelesen werden können.

In der Praxis ergibt sich jedoch insbesondere in Hinblick auf Nikotin das Problem, dass es nicht unüblich ist, dass Personen diese oder andere Substanzen in unterschiedlicher Form zuführen. So ist es beispielsweise nicht unüblich, dass Nutzer einerseits von Zeit zu Zeit ein Pflaster mit der fraglichen Substanz nutzen, jedoch zu anderen Zeiten auch beispielsweise Kaugummis mit der Substanz verwenden. In einem solchen Falle ist die Gefahr groß, dass die betreffende Person den Überblick darüber verliert, wieviel Substanz bereits ihrem Körper zugeführt worden ist.

US 2014/346186 A1 offenbart ein Verdampfersystem zur Nikotinentwöhnung und erfasst Züge/Dosen über Sensoren und verbindet sich drahtlos mit einer Smartphone-App/einem Server zur Protokollierung und Anpassung der Rauchmuster. Das System aggregiert die Nikotinaufnahme über mehrere Sitzungen hinweg und schätzt die Gesamtnikotinmenge (z. B. mg/Tag) mit Grenzwerten/Warnungen ein.

US 2015/061867 A1 beschreibt ein System unterschiedlicher Medikamentenspender zur Überwachung der Medikamenteneinnahme, bezieht sich jedoch nicht auf Nikotin im Speziellen.

### AUFGABE UND LÖSUNG

Die Erfindung wird durch die beigefügten Ansprüche definiert.

Aufgabe der Erfindung ist es, eine Möglichkeit bereitzustellen, mittels derer der oben geschilderten Problemlage begegnet wird und Personen einen Überblick über die Menge der eingenommenen Substanz bewahren können.

Erfindungsgemäß werden hierfür ein System und ein Verfahren vorgeschlagen, die im Folgenden gemeinsam erläutert werden.

Das erfindungsgemäße System und das erfindungsgemäße Verfahren können der Überwachung der Einbringung der pharmakologisch aktiven Substanz Nikotin in den menschlichen Körper dienen. Im Folgenden wird dies erläutert.

Das System umfasst mindestens zwei Spender, die der Abgabe der gleichen Substanz dienen. Die mindestens zwei Spendern sind dabei zur Abgabe der Substanz in unterschiedlicher Applikationsform vorgesehen. Dabei können sich die Applikationsformen darin unterscheiden, ob die Abgabe durch den Spender unmittelbar der Einbringung in den menschlichen Körper dient oder ob sich zunächst ein weiterer Applikationsschritt anschließt, wie beispielsweise die Einnahme oder Aufbringung eines zuvor vom Spender abgegebenen Kaugummis oder eines vom Spender abgegebenen Pflasters.

Die mindestens zwei Spender sind dafür ausgebildet, eine mit der Abgabe von Substanz einhergehenden Handhabung zu erfassen. Diese Handhabung kann unmittelbar jene Handhabung sein, die nicht nur der Abgabe der Substanz aus dem Spender dient, sondern gleichzeitig auch der Einnahme durch den Nutzer. Dies ist beispielsweise bei einem Flüssigkeitsspender gegeben, der in der Lage ist, über einen Sensor die Betätigung zu erfassen, mittels derer Flüssigkeit in den Mund oder die Nase des Nutzers appliziert wird. Die erfasste Handhabung kann jedoch auch eine solche sein, die nicht unmittelbar zur Einnahme führt, sondern zeitlich vor dieser stattfindet. Dies kann beispielsweise gegeben sein, wenn der Spender dem Austrag von Kaugummis oder Tabletten dient. Der Spender erfasst hierbei nicht unmittelbar die Einnahme dieses Kaugummis oder der Tablette, sondern lediglich die Abgabe aus dem Spender oder das Öffnen des Zugangs zu einem Aufnahmeraum des Spenders.

Er wird also in diesem Falle lediglich angenommen, dass der Nutzer in der bestimmungsgemäßen Art das Kaugummi oder die Tablette nach Abgabe aus dem Spender tatsächlich auch einnimmt.

Die mindestens zwei Spender, die somit die Abgabe der Substanz überwachen, sind dafür ausgebildet, die Information über die Abgabe an ein zentrales Überwachungsgerät zu senden. Bei dem zentralen Überwachungsgerät handelt es sich vorzugsweise um ein Gerät, welches ein persönliches Gerät des Nutzers selbst ist, beispielsweise sein Computer. Insbesondere kann es sich um ein Smartphone handeln, dessen Funktionalität durch ein Programm (App) in Hinblick für die im Folgenden noch beschriebenen Funktionen ergänzt ist.

Über eine Datenverbindung, insbesondere eine WLAN- oder Bluetooth-Verbindung, übertragen die mindestens zwei Spender die Daten zur Abgabe der Substanz an das Smartphone oder ein anderweitig ausgestaltetes zentrales Überwachungsgerät. Zu diesem Zwecke weisen die Spender vorzugsweise jeweils ein Funkmodul auf. Vorzugsweise weisen die Spender weiterhin einen integrierten Schaltkreis oder einen Prozessor zur Auswertung von Sensordaten und zur Ansteuerung des Funkmoduls auf. Weiterhin wird es als vorteilhaft angesehen, wenn die Spender über einen Spender verfügen, in dem sie Daten zur erfassten Handhabung ablegen, so dass die Datenübertragung nicht unmittelbar stattfinden muss.

Das Überwachungsgerät ist dafür ausgebildet, die entsprechenden Daten zur Abgabe der Substanz von den mindestens zwei Spendern empfangen.

Diese Daten werden dann erfindungsgemäß in eine Berechnung der Gesamtmenge der abgegebenen Substanz einbezogen. Dies bedeutet, dass das Überwachungsgerät mittels seines Prozessors ausrechnet, wie groß die Gesamtmenge der abgegebenen Substanz der mindestens zwei Spender ist, insbesondere in einem begrenzten Zeitraum, wie dem aktuellen Tag.

Das Überwachungsgerät kann dabei berücksichtigen, welche Menge der Substanz mit jeder Abgabe der Spender einhergeht. So kann beispielsweise berücksichtigt werden, dass ein einzelnes Nikotinpflaster eine Nikotinmenge von beispielsweise 10 mg beinhaltet, während ein Nikotinspray mitjedem Austrag nur 0,5 mg austrägt. Um dies zu berücksichtigen kann bei einer möglichen Gestaltung im Überwachungsgerät die Abgabemenge der Substanz für jeden Spender hinterlegt sein. Demgegenüber von Vorteil ist es jedoch, wenn die Spender selbst nicht oder nicht nur die Anzahl der Abgabevorgänge an das zentrale Überwachungsgerät übermitteln, sondern stattdessen oder auch die damit einhergehende Abgabemenge übermitteln. In einem solchen Fall braucht das Überwachungsgerät nicht spezifisch an den jeweiligen Spender angepasst zu sein.

Die vom Überwachungsgerät errechnete Gesamtmenge der abgegebenen Substanz bezieht sich vorzugsweise auf einen definierten Zeitraum wie beispielsweise auf einen Tag, eine Woche oder einen Monat. Dementsprechend wird vorzugsweise in regelmäßigen Abständen diese zeitintervallbezogene Gesamtmenge wieder auf Null gesetzt bzw. die bei der Errechnung der Gesamtmenge zu berücksichtigenden Abgabehandlungen, die in einem Speicher des Überwachungsgeräts abgespeichert sind, werden bei der Berechnung der Gesamtmenge begrenzt, insbesondere auf solche des aktuellen Tages, der aktuellen Woche oder des aktuellen Monats.

Das Überwachungsgerät, also insbesondere das Smartphone, weist vorzugsweise eine Anzeigeeinrichtung auf, insbesondere in Form eines Displays. Auf dieser Anzeigeeinrichtung wird primär die errechnete Gesamtmenge der von den Spendern abgegebenen Substanz angezeigt, wobei die Darstellung der absoluten Menge selbst vorgesehen sein kann und alternativ oder zusätzlich auch ein Prozentwert, der anzeigt, welchen Anteil an einer für das betreffende Zeitintervall vorgesehenen Gesamtmenge oder Maximalmenge bereits erreicht wurde. Vorzugsweise ist auch eine graphische Darstellung in Form eines Fortschrittsbalkens vorgesehen, die mit einem Blick eine Erfassung des aktuellen Standes gestattet.

Ist eine Maximalmenge oder eine Sollmenge für das entsprechende Zeitintervall, beispielsweise den aktuellen Tag, hinterlegt, so lässt sich ausgehend von der Gesamtmenge der in diesem Zeitintervall bereits abgegebenen Substanz zusätzlich die noch verbleibende Substanzmenge für das Zeitintervall ermitteln.

Vorzugsweise ist vorgesehen, dass das Überwachungsgerät dafür ausgebildet ist, diese verbleibende Substanzmenge anzuzeigen. Um es dem Benutzer besonders einfach zu machen, kann das Überwachungsgerät dafür ausgebildet sein, diese verbleibende Substanzmenge auch noch dahingehend umzurechnen, dass die Zahl der noch verfügbaren Abgabevorgänge spezifisch für jeden Spender des Systems angezeigt wird. So kann beispielsweise in Form von Zahlenwerten ausgegeben werden, wie viele Abgabevorgängen und Nutzungen von Pflastern oder Abgabevorgänge von Spray noch zur Verfügungstünden, wenn die gesamte verbleibende Substanzmenge mit derjeweiligen Applikationsmethode abgegeben würde.

Das Überwachungsgerät ist weiterhin vorzugsweise dafür ausgebildet, visuelle oder akustische Warnmitteilungen abzugeben, wenn die Gesamtmenge im aktuellen Zeitintervall schon zu einem vorgegebenen Anteil, beispielsweise 50%, die Maximalmenge erreicht hat oder wenn die Gesamtmenge die Maximalmenge erreicht oder überschreitet.

Insbesondere die folgenden Arten von Spendern finden vorzugsweise in einem erfindungsgemäßen System Verwendung.

Vorzugsweise ist mindestens einer der Spender als Flüssigkeitsspender zur Abgabe der Substanz in flüssiger Form als unzerstäubter Strahl, als Sprühstrahl oder in Tropfenform ausgebildet. Ein solcher Flüssigkeitsspender verfügt über einen Flüssigkeitsspeicher, dessen Volumen insbesondere vorzugsweise 20 ml oder weniger beträgt. Hier ist die Flüssigkeit, die die aktive Substanz beinhaltet, vor dem Austrag gelagert. Die Flüssigkeit kann im Flüssigkeitsspeicher unter Druck oder drucklos gelagert sein. Der Flüssigkeitsspender verfügt weiterhin über eine Abgabeöffnung, durch die hindurch die Flüssigkeit abgegeben wird. Zur Bewirkung des Austrags ist eine Betätigungshandhabe vorgesehen, also eine kraftbeaufschlagbare Fläche am Spender. Wird diese Betätigungshandhabe betätigt, so wird über eine Pumpe oder über ein Ventil Flüssigkeit aus dem Flüssigkeitsspeicher zur Abgabeöffnung gefördert. Vorzugsweise ist die Austragmenge dabei immer im Wesentlichen identisch. Dies lässt sich beispielsweise über eine Pumpe und deren Pumpkammervolumen gut realisieren.

Die Sensorik zur Erfassung der Abgabe von Flüssigkeit kann in den Spender untrennbar integriert sein, beispielsweise in Form eines Tasters an der Betätigungshandhabe oder eines Durchflussmessers zwischen Flüssigkeitsspeicher und Abgabeöffnung.

Besonders bevorzugt ist allerdings eine Gestaltung, bei der der Flüssigkeitsspender ein Anbaugerät umfasst, welches die Sensorik zur Erfassung des Abgabevorgangs beinhaltet und was von einer Hauptbaugruppe des Spenders, umfassen den Flüssigkeitsspeicher, den Abgabekanal und die Abgabeöffnung trennbar ist, so dass es nach Verbrauch der Flüssigkeit hiervon getrennt werden kann und mit einem neuen Spender bzw. dessen Hauptbaugruppe verbunden werden kann.

Insbesondere kann es sich beim Anbaugerät um ein Gerät handeln, welches am Flüssigkeitsspeicher oder an der Betätigungshandhabe angebracht wird und welches über einen Kraft- oder Drucksensor verfügt, der bei Betätigung der Betätigungshandhabe die Betätigungskraft mittelbar erfasst.

Eine zweite Art eines Spenders, der vorzugsweise Teil eines erfindungsgemäßen Systems ist, ist ein Pflasterspender, der zur Abgabe von Pflastern vorgesehen ist. In diesem kann eine Mehrzahl von Pflastern vorgehalten werden, die jeweils einen mit der Substanz versehenen Speicher aufweisen, der bei Anbringung des Pflasters auf der Haut eines Nutzers an der Haut anliegt.

Hierbei sind insbesondere die folgenden Bauarten von der Erfindung umfasst. Bei einer ersten Bauart weist der Pflasterspender einen Aufnahmeraum auf, in dem die Pflaster gelagert sind. Dieser Aufnahmeraum wird durch ein bewegliches Deckelelement begrenzt, welches bei einer geöffneter Stellung Zugang zum Aufnahmeraum ermöglicht, so dass der Nutzer nach dem Aufklappen ein Pflaster entnehmen kann. Wenngleich die Entnahme selbst durch geeignete Sensorik erfassbar ist, ist es von Vorteil, wenn die Sensorik vorzugsweise lediglich dafür ausgebildet ist, die geöffnete Stellung zu erkennen. Dies kann beispielsweise durch einen einfachen Kraftsensor erreicht werden.

Bei einer solchen Gestaltung wird davon ausgegangen, dass jeder Öffnungsvorgang mit der Entnahme und Verwendung von genau einem Pflaster einhergeht. Entnimmt der Nutzer mehrere Pflaster, so ist dies nicht ohne Weiteres vom System erkennbar. Es kann aber vorgesehen sein, dass das Überwachungsgerät, also insbesondere das hierfür verwendete Smartphone, eine Benutzereingabe zulässt, mit der der Benutzer zu erkennen geben kann, falls er mehr als ein Pflaster entnommen hat oder falls er kein Pflaster entnommen hat.

Eine komplexere alternative Bauweise sieht vor, dass der Pflasterspender eine Betätigungshandhabe aufweist, mittels derer ein einzelnes Pflaster aus dem Pflasterspender entnehmbar ist. In einem solchen Falle erhält der Nutzer somit keinen direkten Zugang zum Aufnahmeraum des Spenders, sondern kann hieraus lediglich einzelne Pflaster entnehmen, wobei dies insbesondere damit einhergeht, dass die Betätigungshandhabe je Pflaster einmal zu betätigen ist. Die Erfassung der Betätigung der Betätigungshandhabe und/oder die Abgabe des Pflasters selbst kann durch passende Sensorik erfasst werden, um diese Information an das zentrale Überwachungsgerät zu übermitteln.

Eine weitere Art eines Spenders, der vorzugsweise Teil eines erfindungsgemäßen Systems ist, ist ein Kaugummispender oder ein Tablettenspender.

Übereinstimmend mit den Ausführungen zum Pflasterspender sind auch hier die beiden beschriebenen Bauweisen möglich, also einerseits eine Bauweise, bei der der Spender einen Aufnahmeraum aufweist, in dem die Kaugummis oder Tabletten gelagert sind und der über ein bewegliches Deckelelement zugänglich ist, und andererseits eine Bauweise, bei der über eine Betätigungshandhabe eine vereinzelte Tablette oder ein vereinzeltes Kaugummi abgegeben wird. Mit Ausnahme der Befüllung und gegebenenfalls der Maße des Aufnahmeraums kann eines solcher Spender grundsätzlich baulich identisch mit dem beschriebenen Pflasterspender sein.

Bei Kaugummispendern und Tablettenspendern wird jedoch eine weitere Bauweise für besonders vorteilhaft gehalten. Bei dieser weiteren Bauweise weist der Kaugummispender bzw. Tablettenspender eine Hülle auf, in der ein Packmittel mit einzelnen Kaugummis oder Tabletten eingeschoben ist. Bei dem Packmittel kann es sich insbesondere um eine Blisterverpackung handeln, die einzelne Kompartments fürjeweils eine Tablette oder ein Kaugummi aufweisen. Dieses Packmittel wird bestimmungsgemäß zum Zwecke der Abgabe einer Tablette oder eines Kaugummis zumindest partiell aus der Hülle herausgezogen.

Ein solcher Kaugummispender und Tablettenspender weist zur Erfassung eines Abgabevorgangs entweder einen Sensor auf, der das Herausziehen des Packmittels gestattet, oder es ist ein Sensor vorgesehen, der das Lösen einer Blockierung erkennt, die dem Herausziehen des Packmittels entgegensteht. So kann beispielsweise ein Druckknopf an einem offenen Ende der Hülle vorgesehen sein, dessen zwei Teile gelöst werden müssen, um das Packmittel dann herausziehen zu können. Der genannte Druckknopf kann zusätzlich einen Sensor darstellen, der es erkennbar macht, dass der Nutzer den Druckknopf geöffnet hat, mutmaßlich, um eine Tablette oder ein Kaugummi zu entnehmen.

Ein vierter Typ eines im erfindungsgemäßen System vorzugsweise vorgesehenen Spenders ist ein Spender zur Abgabe von Flüssigkeitskartuschen. Diese Flüssigkeitskartuschen selbst dienen der Verwendung als Flüssigkeitsspeicher für eine Austragvorrichtung, bei der es sich insbesondere um einen Verdampfer handeln kann, der für die Verwendung von wechselbaren Flüssigkeitskartuschen ausgebildet ist. Ein solcher Verdampfer ist insbesondere vorzugsweise auch Teil des erfindungsgemäßen Systems.

Wie auch schon zum Kaugummi- bzw. Tablettenspender sowie zum Pflasterspender erläutert, ist auch bei diesem Spender zur Abgabe von Flüssigkeitskartuschen vorzugsweise vorgesehen, dass dieser entsprechend einer der dort beschriebenen Bauweisen ausgebildet ist. Er weist also vorzugsweise entweder einen Aufnahmeraum auf, in dem die Flüssigkeitskartuschen gelagert sind und der durch ein bewegliches Deckelelement zugänglich ist, wobei das Öffnen dieses Deckelelements vorzugsweise mittels eines Sensors erfasst wird, oder er verfügt über eine Betätigungshandhabe, die eine vereinzelte Abgabe einer Flüssigkeitskartusche gestattet, wobei die Betätigung der Betätigungshandhabe oder die Abgabe der Flüssigkeitskartusche über einen Sensor erfasst wird.

Die Flüssigkeitskartuschen selbst weisen einen durch eine Wandung begrenzten Speicherbehälter auf, der mit der Substanz in flüssiger Form befüllt ist und der übereinen Entnahmeanschluss verfügt, mittels dessen Sie an einen Einlasskanal der genannten Austragvorrichtung angeschlossen werden, so dass die enthaltene Flüssigkeit zu einem Mundstück der Austragvorrichtung gelangen kann, wo vorzugsweise eine elektrische Erhitzung der Flüssigkeit zum Zwecke der Verdampfung stattfindet.

Alle genannten Arten von Spendern zeichnen sich dadurch aus, dass sie eine Sensorik aufweisen, die die Abgabe der Substanz oder eine mit der Abgabe der Substanz einhergehende Handhabung erfassen. Ein hierbei vorgesehener Sensor kann vorzugsweise sehr einfach ausgebildet sein, beispielsweise in Form eines Kraft- oder Drucksensors. Weiterhin weist einer der Spender des Systems mindestens einen integrierten Schaltkreis auf, der zur Auswertung des Sensors in der Lage ist und der zudem dafür ausgebildet ist, über die vorzugsweise drahtlose Datenschnittstelle mit dem zentralen Überwachungsgerät zu kommunizieren. Diese Datenkommunikation ist vorzugsweise bidirektional vorgesehen und folgt insbesondere vorzugsweise einem WLAN- oder Bluetooth-Standard. Denkbar ist jedoch auch ein hiervon abweichender Funkstandard und gegebenenfalls sogar eine unidirektionale Kommunikation, im Rahmen derer der Spender keinerlei Daten empfängt, sondern lediglich zum Senden ausgebildet ist. Vorzugsweise weisen die Spender eine Energiequelle in Form einer Batterie oder eines Akkumulators auf. Möglich sind jedoch auch Spender, die zur Energieernte ausgebildet sind, also aus der Handhabung zum Zwecke der Abgabe der Substanz elektrische Energie gewinnen, die dann zur Erzeugungdes Funksignals verwendet wird. Wie oben schon beschrieben, weisen die Spender vorzugsweise weiterhin einen Speicher auf, um erfasste Abgabe- oder Entnahmevorgängen zwischenspeichern zu können, insbesondere wenn eine Funkverbindung zum zentralen Überwachungsgerät gerade nicht aufgebaut werden kann.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erfindungsgemäßes System zur Abgabe von Nikotin und zur Überwachung dieser Abgabe in einer Gesamtdarstellung.
Fig. 2 und 3 zeigen Pflasterspender, die Teil des Systems der Fig. 1 sein können.
Fig. 4 zeigt einen Flüssigkeitsspender, der Teil des Systems der Fig. 1 sein kann.
Fig. 5 bis 7 zeigen Tabletten- oder Kaugummispender, die Teil des Systems der Fig. 1 sein können.
Fig. 8 und 9 zeigen Flüssigkeitskartuschenspender, die Teil des Systems der Fig. 1 sein können.

### DETAILLIERTE BESCHREIBUNG DER AUSFÜHRUNGSBEISPIELE

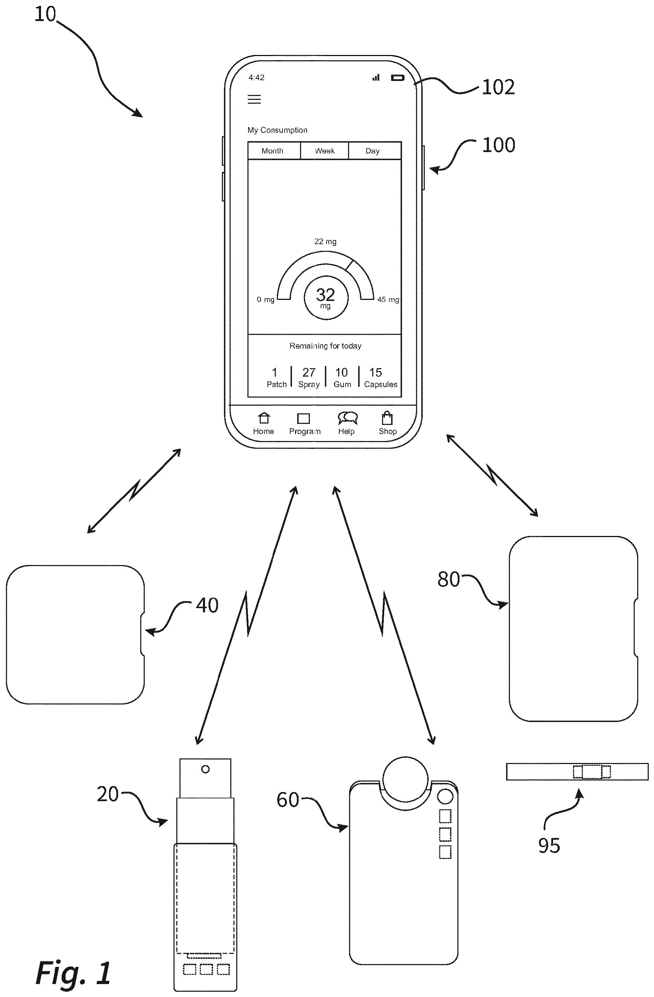

Fig. 1 zeigt ein erfindungsgemäßes System 10 zur Abgabe von Nikotin und zur Überwachung dieser Abgabe in einer Gesamtdarstellung.

Das System umfasst ein zentrales Überwachungsgerät 100, welches vorliegend durch ein Smartphone 100 gebildet wird. Über ein solches Smartphone 100 verfügen heutzutage viele Menschen, so dass üblicherweise dieses bereits vorhandene Gerät vorteilhaft als zentrales Überwachungsgerät nutzbar ist. Das Smartphone 100 verfügt über ein Touch-Display 102, auf dem Informationen dargestellt werden können und Eingaben erfolgen können. Auf dem Smartphone 100 ist ein Programm (App) installiert, welches die Überwachungsfunktionalität des Systems bereitstellt.

Das System 10 umfasst weiterhin eine Mehrzahl von Spendern 20, 40, 60, 80, die jeweils für den Austrag eines Medikaments oder von Nikotin ausgebildet sind.

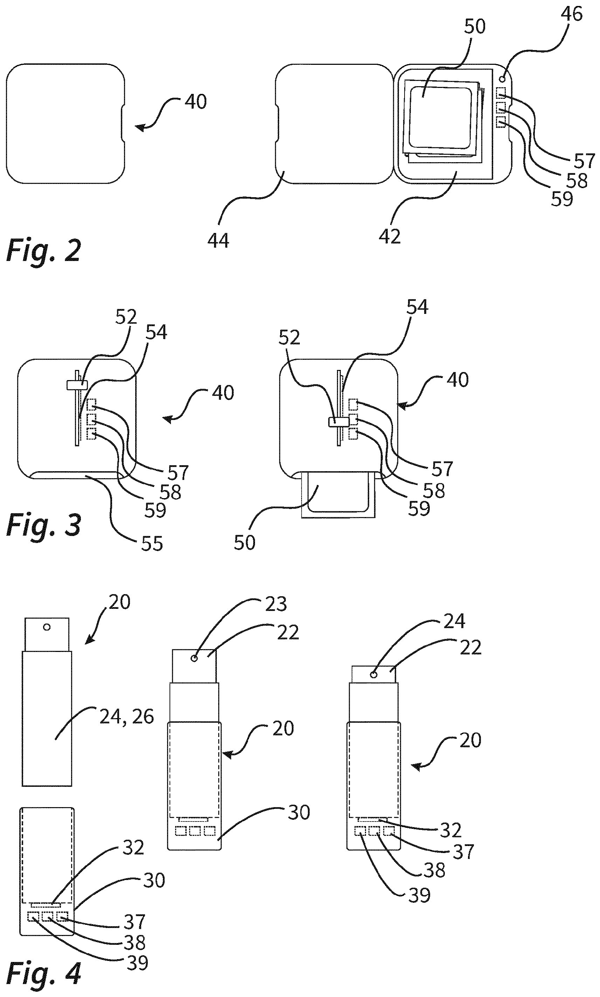

Spender 40 ist ein Pflasterspender 40, der beispielsweise entsprechend einer der Gestaltungen der Fig. 2 und 3 ausgestaltet sein kann. Aus dem Pflasterspender 40 können einzelne Pflaster 50 entnommen oder ausgetragen werden, die anschließend vom Nutzer auf seiner Haut aufgebracht werden, so dass sie über einen längeren Zeitraum Wirkstoff, wie insbesondere Nikotin, abgeben können.

Wird ein Pflaster entnommen oder abgegeben, so wird diese Information vom Pflasterspender 40 über eine drahtlose Funkschnittstelle, wie beispielsweise über Bluetooth Low Energy (BLE) an das Smartphone 100, übermittelt und von der dortigen App verarbeitet. Die Übermittlung kann im einfachsten Falle lediglich die Information der Entnahme oder der Abgabe selbst beinhalten. Ergänzend können jedoch auch Informationen bspw. zur Wirkstoffmenge oder zur Uhrzeit der Abgabe übermittelt werden. Das System 10 kann bei einer einfachen Gestaltung davon ausgehen, dass ein entnommenes oder abgegebenes Pflaster unmittelbar auf der Haut des Nutzers aufgebracht werden. Die App kann sich bei einer anderen Gestaltung jedoch auch per Dialog bestätigen lassen, dass die Aufbringung auf der Haut erfolgt ist.

Spender 20 ist als Flüssigkeitsspender 20 ausgebildet, der durch manuelle Betätigung einer Betätigungshandhabe 22 Flüssigkeit abgibt. In Fig. 4 ist ein hierfür nutzbarer Flüssigkeitsspender 20 genauer dargestellt. Die Flüssigkeit wird bei der Abgabe vorzugsweise unmittelbar in den menschlichen Körper eingebracht und insbesondere unmittelbar auf Schleimhäute appliziert. Insbesondere kann der Flüssigkeitsspender zur Applikation der Flüssigkeit in Augen, Nase oder Mund des Nutzers vorgesehen sein.

Der Flüssigkeitsspender 20 weist einen Flüssigkeitsspeicher 24 auf, gegenüber dem die Betätigungshandhabe 22 zur Abgabe von Flüssigkeit niederdrückbar ist. Das Niederdrücken der Betätigungshandhabe 22 und damit mittelbar der hierdurch bewirkte Austrag durch eine Abgabeöffnung an der Betätigungshandhabe 22 kann durch entsprechende Sensorik erfasst werden. Die Information des Austrags wird, gegebenenfalls ergänzt um zusätzliche Daten wie die Austragmenge und/oder die Uhrzeit des Austrags, an das Smartphone 100 und die dortige App übermittelt.

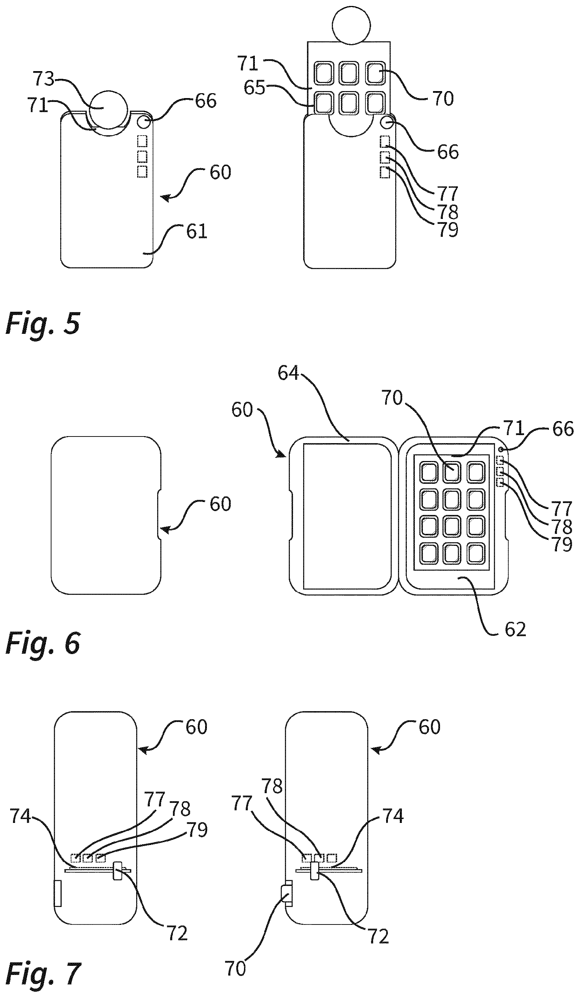

Der Spender 60 ist ein Kaugummispender 60 oder Tablettenspender 60, der beispielsweise entsprechend einer der Fig. 5 bis 7 ausgebildet sein kann. Aus dem Spender 60 können einzelne Kaugummis 70 oder Tabletten 70 entnommen oder ausgetragen werden, die anschließend vom Benutzer geschluckt bzw. gekaut werden, so dass der enthaltene Wirkstoff vom Körper aufgenommen wird.

Wird ein Kaugummi oder eine Tabelle entnommen oder abgegeben, so wird diese Information vom Spender60 wiederum über eine drahtlose Funkschnittstelle an das Smartphone 100 übermittelt und von der dortigen App verarbeitet. Entweder geht die App dabei davon aus, dass die Verwendung des Kaugummis oder der Tablette 70 unmittelbar erfolgt ist, oder die App stellt einen Dialog auf dem Display 102 dar, um eine Bestätigung des Nutzers betreffend die Einnahme des Kaugummis oder der Tablette zu erbitten.

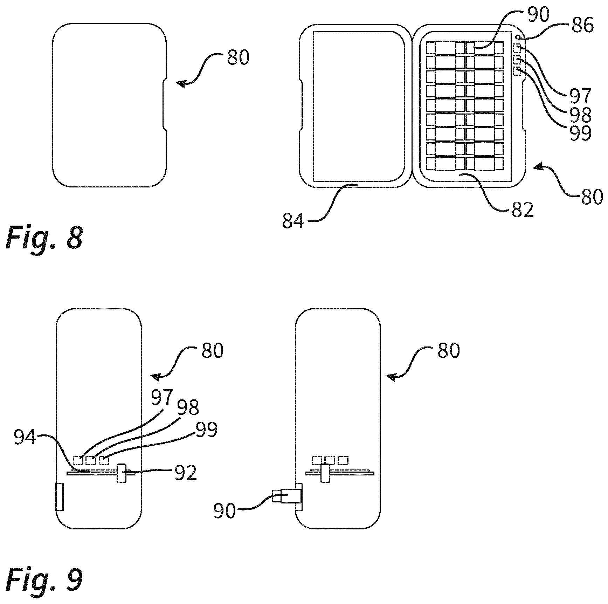

Der Spender 80 ist ein Spender für Flüssigkeitskartuschen 90, der beispielsweise wie in Fig. 8 und 9 dargestellt ausgebildet sein kann. Die Flüssigkeitskartuschen 90 werden nach Entnahme aus dem Spender 80 bestimmungsgemäß mittels separater Austragvorrichtungen 95, insbesondere in Artvon Verdampfern, zur Anwendung gebracht, wobei der Nutzer den insbesondere verdampften Inhalt über eine solche Austragvorrichtung einatmen bzw. inhalieren kann.

Wird eine Flüssigkeitskartusche 90 aus dem Spender 80 entnommen oder abgegeben, so wird diese Information vom Spender 80 wiederum über eine drahtlose Funkschnittstelle an das Smartphone 100 übermittelt und von der dortigen App verarbeitet. Entweder geht die App dabei davon aus, dass die Flüssigkeitskartusche 90 unmittelbar in die Austragvorrichtung 95 eingesetzt und verwendet wird, oder die App erfragt den Zeitpunkt der Verwendung über das Display 102.

Das zentrale Überwachungsgerät 100, vorliegend durch ein Smartphone 100 gebildet, erhält somit von verschiedenen Spendern 20, 40, 60, 80 Informationen zur Abgabe von Wirkstoff, gegebenenfalls ergänzt um die konkret abgegebene Wirkstoffmenge.

Das zentrale Überwachungsgerät 100 kann diese Informationen getrennt nach Spender 20, 40, 60, 80 speichern und die jeweiligen Wirkstoffmengen dann zu einer Summe, beispielsweise einer Tagessumme, aufaddieren, um den Gesamtkonsum des Medikaments bzw. von Nikotin zu errechnen. Der entsprechende Wert, im Ausführungsbeispiel zum gegenwärtigen Zeitpunkt 32 Milligramm, wird auf dem Display 102 ausgegeben, so dass der Nutzer hierüber informiert ist. Weiterhin zeigt das Display an, dass die vorgegebene Maximalmenge je Tag vorliegend bei 45 Milligramm liegt und wie viele Pflaster 50, Kaugummis 70, Flüssigkeitskartuschen 90 oder Sprühstöße durch den Flüssigkeitsspender für die verbleibenden 13 Milligramm zulässig sind.

Der Nutzer kann im Falle der Nutzung für Nikotin somit in sehr leichter Form einen Überblick bewahren und sich die bis zur Maximalmenge verbleibende Restmenge in individuell gewünschter Weise einteilen.

Anhand der weiteren Figuren sind exemplarisch mögliche Spender etwas detaillierter dargestellt und erläutert.

Fig. 2 zeigt eine erste Variante eines Pflasterspenders40 in geschlossenem und geöffnetem Zustand. Der Pflasterspender 40 verfügt über einen Aufnahmeraum 42, in dem mehrere Pflaster 50 gelagert werden. Der Aufnahmeraum kann durch Aufschwenken eines Deckelelements44von außen zugänglich gemacht werden, so dass dann ein Pflaster 50 entnommen werden kann. Das Öffnen des Spenders 40 wird über einen Sensor 46, beispielsweise einen Taster, erfasst, und mit einem Prozessor 57 und einem Funkmodul 58, welche von einer Batterie 59 gespeist werden, zum Überwachungsgerät 100 gesendet.

Fig. 3 zeigt eine zweite Variante eines Pflasterspenders 40. Dieser weist ebenfalls einen Aufnahmeraum für Pflaster 50 auf. Dieser ist jedoch nicht unmittelbar zugänglich. Stattdessen verfügt der Spender 40 über einen Ausgabeschlitz 55, durch den vereinzelt Pflaster 50 mittels einer Betätigungshandhabe 52 herausgeschoben werden können. Mittels eines Sensors 54, beispielsweise in Art eines Potentiometers, kann dies erfasst werden. Wiederum wird die Information des Austrags über einen Prozessor 57 und ein Funkmodul58 an das Überwachungsgerät 100 gesendet.

Fig.4 zeigt eine Variante eines Flüssigkeitsspenders 20. Dieser weist in der schon beschriebenen Weise einen Flüssigkeitsspeicher 24 auf, gegenüber dem eine Betätigungshandhabe 22 niedergedrückt werden kann, um eine interne Pumpe zu betätigen oder ein Abgabeventil zu öffnen. Die Flüssigkeit kann das in Form eines unzerstäubten Jets oder eines Sprühstrahls oder aber auch in Form von Einzeltropfen durch eine Abgabeöffnung 23 abgegeben werden. Insbesondere bevorzugt sind Gestaltungen, bei denen hierbei eine vorgegebene Flüssigkeitsmenge ausgetragen wird.

Obwohl eine denkbare technische Bauweise vorsieht, dass ein unmittelbar integrierter Sensor diese Betätigung erfasst, ist es bevorzugt, dass der Flüssigkeitsspender 20 ein Anbaugerät 30 aufweist, welches für die Funktionalität des Austrags selbst entbehrlich ist und welches zusätzlich angebracht wird, um die Betätigung erfassen zu können. Vorliegend weist das Anbaugerät 30 einen rohrförmigen Abschnitt auf, in den ein Gehäuseteil 26 des Flüssigkeitsspender 20 eingeschoben wird. Am Boden dieses rohrförmigen Abschnitts ist ein Kraftsensor 32 vorgesehen, der bei Kraftbeaufschlagung der Handhabe 22 die entsprechende Kraft erfasst und hierdurch erkennen kann, ob eine Betätigung und mutmaßlich ein Austrag stattgefunden hat. Sofern eine Betätigung und ein Austragvorgang stattgefunden haben, wird dies durch den Prozessor 37 und das Funkmodul 38 an das Überwachungsgerät 100 übermittelt.

Fig. 5 bis 7 zeigen unterschiedliche Varianten eines Kaugummispenders 60. Bei der Gestaltung der Fig. 5 weist dieser eine Hülle 61, beispielsweise aus Kunstleder, auf, in die eine Blisterverpackung 71 mit einer Vielzahl von Kaugummis 70 eingeschoben ist. Die Blisterverpackung 71 kann über ein Zugelement 73 aus der Hülle 61 herausgezogen werden, allerdings erst, wenn ein Druckknopf 66 zuvor gelöst wurde. Dieser Druckknopf 66 ist gleichzeitig ein Sensor, so dass der Prozessor 77 erfassen kann, dass der Druckknopf gelöst wird. Dies wird als Signal dafür gewertet, dass der Nutzer die Blisterverpackung 71 herauszieht und ein Kaugummi 70 entnimmt und verwendet. Diese Information wird mittels des Funkmoduls 78 an das Überwachungsgerät übermittelt.

Die Varianten des Kaugummispenders der Fig. 6 und 7 entsprechen im Wesentlichen den Pflasterspendern der Fig. 2 und 3. Wie zur Fig. 2 beschrieben, stellt die Gestaltung der Fig. 6 einen Spender mit einem Aufnahmeraum 62 dar, in dem in diesem Falle Einzelkaugummis gelagert werden, ggf. in einer Blisterverpackung 71. Das Öffnen des Deckelelements 64 wird mittels eines Sensors 66 erfasst und über das Funkmodul 78 an das Überwachungsgerät 100 gemeldet. Bei der Gestaltung der Fig. 7 handelt es sich entsprechend Fig. 3 um einen Spender zur Einzelabgabe, in diesem Falle von Kaugummis 70, die mittels einer Betätigungshandhabe 72 einzeln abgegeben werden können, um vom Nutzer konsumiert zu werden. Die Betätigung der Betätigungshandhabe 72 wird über einen geeigneten Sensor 74 erfasst und der damit einhergehende Austrag an das Überwachungsgerät 100 übermittelt.

Die Fig. 8 und 9 zeigen Spender 80 für Flüssigkeitskartuschen 90. Wie oben bereits beschrieben, handelt es sich um Kartuschen, die in Austragvorrichtungen, wie Verdampfern, eingesetzt werden, um die darin enthaltene Flüssigkeit austragen zu können.

Die Spender der Fig. 8 und 9 entsprechen im Wesentlichen den Pflasterspendern der Fig. 2 und 3. Der Spender der Fig. 8 ermöglicht den Zugang zu einem Aufnahmeraum 82, in dem die Kartuschen gelagert sind und aus dem sie entnommen werden können, wobei der Sensor 86 das Öffnen des Deckelelements 84 erfasst, so dass dies über den Prozessor 97 und das Funkmodul 98 an das Überwachungsgerät 100 gesendet werden kann. Der Spender der Fig. 9 erlaubt wiederum den vereinzelten Austrag der Kartuschen 90, wobei der Austrag durch einen Sensor 94 an der Betätigungshandhabe 92 erfasst wird und an das Überwachungsgerät 100 gemeldet wird.

## Patentansprüche

1. System (10) zur Überwachung der Einnahme von Nikotin mit den folgenden Merkmalen:
a. das System (10) weist mindestens zwei Spender (20, 40, 60, 80) zur Abgabe von Nikotin auf, wobei die beiden Spender (20,40, 60, 80) zur Abgabe von Nikotin in unterschiedlicher Applikationsform ausgebildet sind, und
b. das System (10) weist ein mit den mindestens zwei Spendern (20, 40, 60, 80) durch eine Datenverbindung verbundenes Überwachungsgerät (100) auf, und
c. die beiden Spender (20, 40, 60, 80) sind dafür ausgebildet, eine mit der Abgabe von Nikotin einhergehenden Handhabung zu erfassen und in Reaktion hierauf Daten zur Abgabe des Nikotins an das Überwachungsgerät (100) zu senden, und
d. das Überwachungsgerät (100) ist dafür ausgebildet, die Daten zur Abgabe des Nikotins von den mindestens zwei Spenders (20, 40, 60, 80) zu empfangen und eine Gesamtmenge des abgegebenen Nikotins zu berechnen, und
e. mindestens einer der Spender (40) ist als Pflasterspender (40) zur Abgabe von Pflastern (50) vorgesehen, wobei der Pflasterspender (40) ist mit einer Mehrzahl von Pflastern (50) befüllt, die jeweils einen mit Nikotin versehenen Speicher aufweisen, der bei Anbringung des Pflasters auf der Haut eines Nutzers an der Haut anliegt,
und/oder
mindestens einer der Spender (60) ist als Kaugummispender (60) ausgebildet, wobei der Kaugummispender (60) mit einer Mehrzahl von Kaugummis (70) befüllt ist, die jeweils Nikotin enthalten.

2. System (10) nach Anspruch 1 mit dem folgenden weiteren Merkmal:
a. das Überwachungsgerät (100) weist eine Anzeigeeinrichtung (102) auf und ist dafür ausgebildet, die Gesamtmenge des abgegebenen Nikotins auf der Anzeigeeinrichtung (102) darzustellen.

3. System (10) nach einem der vorstehenden Ansprüche mit dem folgenden weiteren Merkmal:
a. das Überwachungsgerät (100) ist dafür ausgebildet, die Gesamtmenge über einen begrenzten Zeitraum zu berechnen, insbesondere über einen Tag.

4. System (10) nach einem der vorstehenden Ansprüche mit mindestens einem der folgenden weiteren Merkmale:
a. das Überwachungsgerät (100) ist dafür ausgebildet, die Gesamtmenge mit einer vorgegebenen Maximalmenge zu vergleichen und eine Warnmitteilung auszugeben, wenn die Gesamtmenge erreicht und/oder überschritten wird, und/oder
b. das Überwachungsgerät (100) ist dafür ausgebildet, die Gesamtmenge mit einer vorgegebenen Maximalmenge zu vergleichen und zu errechnen, welche Austragmenge am aktuellen Tag, in der aktuellen Woche oder im aktuellen Monat noch zur Verfügung steht.

5. System (10) nach einem der vorstehenden Ansprüche mit dem folgenden weiteren Merkmal:
a. mindestens einer der Spender (20) ist als Flüssigkeitsspender (20) zur Abgabe von Nikotin in flüssiger Form als unzerstäubter Strahl, als Sprühstrahl oder in Tropfenform ausgebildet,
insbesondere mit mindestens einem der folgenden zusätzlichen Merkmale:
b. der mindestens eine Flüssigkeitsspender (20) weist eine Betätigungshandhabe (22) auf und ist zur Erfassung einer Betätigung der Betätigungshandhabe (22) ausgebildet, und/oder
c. der mindestens eine Flüssigkeitsspender (20) weist ein Anbaugerät (30) auf, welches mit einem Gehäuseteil (26) des Flüssigkeitsspenders (20) lösbar gekoppelt ist und zur Erfassung der Handhabung des Flüssigkeitsspenders (20) ausgebildet ist, und/oder
d. der mindestens eine Flüssigkeitsspender (20) weist einen Flüssigkeitsspeicher (24) auf, dessen Inhalt Nikotin umfasst, und/oder
e. der mindestens eine Flüssigkeitsspender (20) ist als Pumpspender ausgebildet.

6. System (10) nach einem der vorstehenden Ansprüche mit mindestens einem der folgenden zusätzlichen Merkmale:
a. der Pflasterspender (40) weist einen Aufnahmeraum (42) zur Aufnahme der Pflaster (50) auf, wobei ein bewegliches Deckelelement (44) vorgesehen ist, welches in einer geöffneten Stellung Zugang zum Aufnahmeraum (42) und die Entnahme eines Pflasters (50) gestattet, wobei der Pflasterspender (40) vorzugsweise einen Sensor (46) zur Erfassung der geöffneten Stellung aufweist, und/oder
b. der Pflasterspender (40) weist eine Betätigungshandhabe (52) auf, mittels derer ein einzelnes Pflaster (50) aus dem Pflasterspender (40) entnehmbar ist, wobei der Pflasterspender (40) vorzugsweise einen Sensor (54) zur Erfassung der Betätigung der Betätigungshandhabe (52) aufweist.

7. System (10) nach einem der vorstehenden Ansprüche mit mindestens einem der folgenden zusätzlichen Merkmale :
a. mindestens einer der Spender (60) ist als Tablettenspender (60) ausgebildet, und/oder
b. der Kaugummispender (60) bzw. Tablettenspender (60) weist eine Hülle (61) auf, in der ein Packmittel (71) mit einzelnen Kaugummis (70) oder Tabletten (70) enthalten ist, wobei das Packmittel (71) zur Entnahme eines Kaugummis (70) oder einer Tablette (70) aus der Hülle (61) herausziehbar ist und wobei vorzugsweise das Herausziehen des Packmittels (71) oder das Lösen einer Blockierung, um das Packmittel (71) herausziehen zu können, mittels eines Sensors (66) erfassbar ist, und/oder
c. der Kaugummispender (60) bzw. Tablettenspender (60) weist einen Aufnahmeraum (62) zur Aufnahme der Kaugummis (70) oder Tabletten (70) auf, wobei ein bewegliches Deckelelement (64) vorgesehen ist, welches in einer geöffneten Stellung Zugang zum Aufnahmeraum (62) und die Entnahme eines Kaugummis (70) bzw. einer Tablette (70) gestattet, wobei der Kaugummispender (60) bzw. Tablettenspender (60) vorzugsweise einen Sensor (66) zur Erfassung der geöffneten Stellung aufweist, und/oder
d. der Kaugummispender (60) bzw. Tablettenspender (60) weist eine Betätigungshandhabe (72) auf, mittels derer ein einzelner Kaugummi (70) bzw. eine einzelne Tablette (70) aus dem Kaugummispender (60) oder Tablettenspender (60) entnehmbar ist, wobei der Kaugummispender (60) bzw. Tablettenspender (60) vorzugsweise einen Sensor (74) zur Erfassung der Betätigung der Betätigungshandhabe (72) aufweist.

8. System (10) nach einem der vorstehenden Ansprüche mit dem folgenden weiteren Merkmal:
a. mindestens einer der Spender (80) ist zur Abgabe von Flüssigkeitskartuschen (90) ausgebildet, wobei diese Flüssigkeitskartuschen (90) zur Kopplung an eine Austragvorrichtung (95) vorgesehen sind.
vorzugsweise mit mindestens einem der folgenden zusätzlichen Merkmale:
b. der Spender (80) weist einen Aufnahmeraum (82) zur Aufnahme der Flüssigkeitskartuschen (90) auf, wobei ein bewegliches Deckelelement (84) vorgesehen ist, welches in einer geöffneten Stellung Zugang zum Aufnahmeraum (82) und die Entnahme einer Flüssigkeitskartusche (90) gestattet, wobei der Spender (80) vorzugsweise einen Sensor (86) zur Erfassung der geöffneten Stellung aufweist, und/oder
c. der Spender (80) weist eine Betätigungshandhabe (92) auf, mittels derer eine einzelne Flüssigkeitskartusche (90) aus dem Spender (80) entnehmbar ist, wobei der Spender (80) vorzugsweise einen Sensor (94) zur Erfassung der Betätigung der Betätigungshandhabe (92) aufweist.

9. System (10) nach einem der vorstehenden Ansprüche mit den folgenden weiteren Merkmalen:
a. mindestens einer der mindestens zwei Spender (20, 40, 60, 80) ist als Pflasterspender (40) vorgesehen und mindestens einer der mindestens zwei Spender ist als Flüssigkeitsspender (20) ausgebildet, und/oder
b. mindestens einer der mindestens zwei Spender (20, 40, 60, 80) ist als Pflasterspender (40) vorgesehen und mindestens einer der mindestens zwei Spender ist als Kaugummispender (60) oder Tablettenspender (60) ausgebildet, und/oder
c. mindestens einer der mindestens zwei Spender (20, 40, 60, 80) ist als Pflasterspender (40) vorgesehen und mindestens einer der mindestens zwei Spender ist als Spender (80) zur Abgabe von Flüssigkeitskartuschen (90) ausgebildet, und/oder
d. mindestens einer der mindestens zwei Spender (20, 40, 60, 80) ist als Flüssigkeitsspender (20) vorgesehen und mindestens einer der mindestens zwei Spender ist als Kaugummispender (60) oder Tablettenspender (60) ausgebildet, und/oder
e. mindestens einer der mindestens zwei Spender (20, 40, 60, 80) ist als Flüssigkeitsspender (20) vorgesehen und mindestens einer der mindestens zwei Spender ist als Spender (80) zur Abgabe von Flüssigkeitskartuschen (90) ausgebildet, und/oder
f. mindestens einer der mindestens zwei Spender (20, 40, 60, 80) ist als Kaugummispender (60) oder Tablettenspender (60) vorgesehen und mindestens einer der mindestens zwei Spender ist als Spender (80) zur Abgabe von Flüssigkeitskartuschen (90) ausgebildet.

10. System (10) nach einem der vorstehenden Ansprüche mit mindestens einem der folgenden weiteren Merkmale:
a. die Datenverbindung ist eine WLAN- oder Bluetooth-Datenverbindung oder eine GSM-, 3G-, 4G- oder 5G-Mobilfunkverbindung, und/oder
b. das Überwachungsgerät (100) und mindestens einer der Spender (20, 40, 60, 80) sind für einen Kopplungsvorgang ausgebildet, im Zuge dessen ein eindeutiges Kennzeichen am Spender durch das Smartphone eingelesen wird, und/oder
c. das Überwachungsgerät (100) wird durch einen Computer, insbesondere ein Smartphone (100) oder ein Tablet-Computer, gebildet.

11. Verfahren zur Überwachung der Einnahme von Nikotin aus mehreren Spendern (20, 40, 60, 80) in unterschiedlicher Applikationsform mit den folgenden Merkmalen:
a. an mindestens zwei Spendern (20, 40, 60, 80), die zur Abgabe des Nikotins in unterschiedlichen Applikationsformen ausgebildet sind, wird die jeweilige Handhabung des Spenders (20, 40, 60, 80) über Sensoren erfasst, und
b. die mindestens zwei Spender (20, 40, 60, 80) senden in Reaktion hierauf Daten zur Abgabe des Nikotins an ein Überwachungsgerät (100), und
c. das Überwachungsgerät (100) empfängt die Daten und berechnet auf Basis dessen eine Gesamtmenge des durch die mindestens zwei Spender (20, 40, 60, 80) abgegebenen Nikotins, und
d. mindestens einer der Spender (40) ist als Pflasterspender (40) zur Abgabe von Pflastern (50) vorgesehen, wobei der Pflasterspender (40) ist mit einer Mehrzahl von Pflastern (50) befüllt, die jeweils einen mit Nikotin versehenen Speicher aufweisen, der bei Anbringung des Pflasters auf der Haut eines Nutzers an der Haut anliegt,
und/oder
mindestens einer der Spender (60) ist als Kaugummispender (60) ausgebildet, wobei der Kaugummispender (60) mit einer Mehrzahl von Kaugummis (70) befüllt ist, die jeweils Nikotin enthalten.

12. Verfahren nach Anspruch 11 mit dem folgenden weiteren Merkmal:
a. mindestens einer der Spender (20, 40, 60, 80) erfasst eine Handhabung des Spenders, die dem Öffnen eines Aufnahmeraums (42, 62, 62, 82) dient, und sendet in Reaktion hierauf Daten an das Überwachungsgerät.

13. Verfahren nach Anspruch 11 oder 12 mit dem folgenden weiteren Merkmal:
a. mindestens einer der Spender (20, 40, 60, 80) erfasst eine Handhabung des Spenders, die den Austrag des Nikotins verursacht, und sendet in Reaktion hierauf Daten an das Überwachungsgerät,
insbesondere mit mindestens einem der folgenden zusätzlichen Merkmale:
b. der Spender erfasst mittels eines Sensors die Kraftbeaufschlagung und/oder Verlagerung einer Betätigungshandhabe, und/oder
c. der Austrag erfolgt in flüssiger Form, insbesondere als unzerstäubter Jet, als Sprühstrahl oder als Tropfen, oder
d. der Austrag erfolgt in Form eines Pflasters (50), eines Kaugummis (70) oder einer Tablette, die nach dem Austrag von einem Benutzer eingenommen bzw. aufgebracht werden, oder
e. der Austrag erfolgt in Form einer Flüssigkeitskartusche (90), die nach dem Austrag in einen Applikator eingesetzt wird.

## Claims

1. System (10) for monitoring the intake of nicotine, having the following features:
a. the system (10) has at least two dispensers (20, 40, 60, 80) for delivering nicotine, wherein the two dispensers (20, 40, 60, 80) are designed to deliver nicotine in a different application form, and
b. the system (10) has a monitoring device (100) connected to the at least two dispensers (20, 40, 60, 80) by a data link, and
c. the two dispensers (20, 40, 60, 80) are designed to detect a handling associated with the delivery of nicotine and, in reaction hereto, to transmit data relating to the delivery of the nicotine to the monitoring device (100), and
d. the monitoring device (100) is designed to receive the data relating to the delivery of the nicotine from the at least two dispensers (20, 40, 60, 80) and to calculate a total quantity of the delivered nicotine, and
e. at least one of the dispensers (40) is provided as a patch dispenser (40) for delivering patches (50), wherein the patch dispenser (40) is filled with a plurality of patches (50), which each have a store provided with nicotine which, when the patch is applied to the skin of a user, rests on the skin,
and/or
at least one of the dispensers (60) is designed as a chewing gum dispenser (60), wherein the chewing gum dispenser (60) is filled with a plurality of pieces of chewing gum (70), which each contain nicotine.

2. System (10) according to Claim 1, having the following further feature:
a. the monitoring device (100) has an indicating device (102) and is designed to display the total quantity of the delivered nicotine on the indicating device (102).

3. System (10) according to either of the preceding claims, having the following further feature:
a. the monitoring device (100) is designed to calculate the total quantity over a limited time period, in particular over one day.

4. System (10) according to one of the preceding claims, having at least one of the following further features:
a. the monitoring device (100) is designed to compare the total quantity with a predefined maximum quantity and to output a warning message if the total quantity is reached and/or exceeded, and/or
b. the monitoring device (100) is designed to compare the total quantity with an envisaged maximum quantity and to calculate what delivery quantity is still available on the current day, in the current week or in the current month.

5. System (10) according to one of the preceding claims, having the following further feature:
a. at least one of the dispensers (20) is designed as a liquid dispenser (20) for delivering nicotine in liquid form as a non-atomized jet, as a spray jet or in droplet form,
in particular having at least one of the following additional features:
b. the at least one liquid dispenser (20) has an actuating handle (22) and is designed to detect an actuation of the actuating handle (22), and/or
c. the at least one liquid dispenser (20) has an attachment (30), which is detachably coupled to a housing part (26) of the liquid dispenser (20) and is designed to detect the handling of the liquid dispenser (20), and/or
d. the at least one liquid dispenser (20) has a liquid store (24), the content of which comprises nicotine, and/or
e. the at least one liquid dispenser (20) is designed as a pump dispenser.

6. System (10) according to one of the preceding claims, having at least one of the following additional features:
a. the patch dispenser (40) has a receiving chamber (42) for receiving the patches (50), wherein a movable cover element (44) is provided which, in an opened position, permits access to the receiving chamber (42) and the removal of a patch (50), wherein the patch dispenser (40) preferably has a sensor (46) for detecting the opened position, and/or
b. the patch dispenser (40) has an actuating handle (52), by means of which an individual patch (50) can be removed from the patch dispenser (40), wherein the patch dispenser (40) preferably has a sensor (54) for detecting the actuation of the actuating handle (52).

7. System (10) according to one of the preceding claims, having at least one of the following additional features:
a. at least one of the dispensers (60) is designed as a tablet dispenser (60), and/or
b. the chewing gum dispenser (60) or tablet dispenser (60) has a sleeve (61), into which a packaging means (71) with individual pieces of chewing gum (70) or tablets (70) is contained, wherein the packaging means (71) can be drawn out of the sleeve (61) to remove a piece of chewing gum (70) or a tablet (70), and wherein the pulling out of the packaging means (71) or the release of a blockage in order to be able to pull out the packaging means (71) can preferably be detected by means of a sensor (66), and/or
c. the chewing gum dispenser (60) or tablet dispenser (60) has a receiving chamber (62) for receiving the chewing gum (70) or tablets (70), wherein a movable cover element (64) is provided which, in an opened position, permits access to the receiving chamber (62) and the removal of a piece of chewing gum (70) or a tablet (70), wherein the chewing gum dispenser (60) or tablet dispenser (60) preferably has a sensor (66) for detecting the opened position, and/or
d. the chewing gum dispenser (60) or tablet dispenser (60) has an actuating handle (72), by means of which an individual piece of chewing gum (70) or an individual tablet (70) can be removed from the chewing gum dispenser (60) or tablet dispenser (60), wherein the chewing gum dispenser (60) or tablet dispenser (60) preferably has a sensor (74) for detecting the actuation of the actuating handle (72).

8. System (10) according to one of the preceding claims, having the following further feature:
a. at least one of the dispensers (80) is designed to deliver liquid cartridges (90), wherein these liquid cartridges (90) are provided to be coupled to a delivery device (95),
preferably having at least one of the following additional features:
b. the dispenser (80) has a receiving chamber (82) for receiving the liquid cartridges (90), wherein a movable cover element (84) is provided which, in an opened position, permits access to the receiving chamber (82) and the removal of a liquid cartridge (90), wherein the dispenser (80) preferably has a sensor (86) for detecting the opened position, and/or
c. the dispenser (80) has an actuating handle (92), by means of which an individual liquid cartridge (90) can be removed from the dispenser (80), wherein the dispenser (80) preferably has a sensor (94) for detecting the actuation of the actuating handle (92).

9. System (10) according to one of the preceding claims, having the following further features:
a. at least one of the at least two dispensers (20, 40, 60, 80) is provided as a patch dispenser (40) and at least one of the at least two dispensers is designed as a liquid dispenser (20), and/or
b. at least one of the at least two dispensers (20, 40, 60, 80) is provided as a patch dispenser (40) and at least one of the at least two dispensers is designed as a chewing gum dispenser (60) or tablet dispenser (60), and/or
c. at least one of the at least two dispensers (20, 40, 60, 80) is provided as a patch dispenser (40) and at least one of the at least two dispensers is designed as a dispenser (80) for delivering liquid cartridges (90), and/or
d. at least one of the at least two dispensers (20, 40, 60, 80) is provided as a liquid dispenser (20) and at least one of the at least two dispensers is designed as a chewing gum dispenser (60) or tablet dispenser (60), and/or
e. at least one of the at least two dispensers (20, 40, 60, 80) is provided as a liquid dispenser (20) and at least one of the at least two dispensers is designed as a dispenser (80) for delivering liquid cartridges (90), and/or
f. at least one of the at least two dispensers (20, 40, 60, 80) is provided as a chewing gum dispenser (60) or tablet dispenser (60) and at least one of the at least two dispensers is designed as a dispenser (80) for delivering liquid cartridges (90).

10. System (10) according to one of the preceding claims, having at least one of the following further features:
a. the data link is a WLAN or Bluetooth data link or a GSM, 3G, 4G or 5G mobile radio link, and/or
b. the monitoring device (100) and at least one of the dispensers (20, 40, 60, 80) are designed for a coupling operation, in the course of which an unambiguous identifier on the dispenser is read by the smartphone, and/or
c. the monitoring device (100) is formed by a computer, in particular a smartphone (100) or a tablet computer.

11. Method for monitoring the intake of nicotine from a plurality of dispensers (20, 40, 60, 80) in a different application forms, having the following features:
a. on at least two dispensers (20, 40, 60, 80) which are designed to deliver the nicotine in different application forms, the respective handling of the dispenser (20, 40, 60, 80) is detected via sensors, and
b. the at least two dispensers (20, 40, 60, 80) send data relating to the delivery of the nicotine to a monitoring device (100) in reaction thereto, and
c. the monitoring device (100) receives the data and, on the basis of the same, calculates a total quantity of the nicotine delivered by the at least two dispensers (20, 40, 60, 80), and
d. at least one of the dispensers (40) is provided as a patch dispenser (40) for delivering patches (50), wherein the patch dispenser (40) is filled with a plurality of patches (50), which each have a store provided with nicotine which, when the patch is applied to the skin of a user, rests on the skin,
and/or
at least one of the dispensers (60) is designed as a chewing gum dispenser (60), wherein the chewing gum dispenser (60) is filled with a plurality of pieces of chewing gum (70), which each contain nicotine.

12. Method according to Claim 11, having the following further feature:
a. at least one of the dispensers (20, 40, 60, 80) detects a handling of the dispenser which is used to open a receiving chamber (42, 62, 62, 82) and sends data to the monitoring device as a reaction thereto.

13. Method according to Claim 11 or 12, having the following further feature:
a. at least one of the dispensers (20, 40, 60, 80) detects a handling of the dispenser which causes the delivery of the nicotine, and sends data to the monitoring device as a reaction thereto,
in particular having at least one of the following additional features:
b. by means of a sensor, the dispenser detects the application of force and/or displacement of an actuating handle, and/or
c. the delivery is carried out in liquid form, in particular as a non-atomized jet, as a spray jet or as droplets, or
d. the delivery is carried out in the form of a patch (50), a piece of chewing gum (70) or a tablet, which are ingested or applied by a user following the delivery, or
e. the delivery is carried out in the form of a liquid cartridge (90), which is inserted into an applicator following the delivery.

## Revendications

1. Système (10) de surveillance de la consommation de nicotine, ayant les caractéristiques suivantes :
a. le système (10) possède au moins deux distributeurs (20, 40, 60, 80) destinés à délivrer de la nicotine, les deux distributeurs (20, 40, 60, 80) étant configurés pour délivrer de la nicotine sous différentes formes d'application, et
b. le système (10) possède un appareil de surveillance (100) qui est connecté aux au moins deux distributeurs (20, 40, 60, 80) par l'intermédiaire d'une liaison de données, et
c. les deux distributeurs (20, 40, 60, 80) sont configurés pour détecter une manipulation associée à la délivrance de nicotine et, en réaction à celle-ci, pour envoyer des données relatives à la délivrance de nicotine à l'appareil de surveillance (100), et
d. l'appareil de surveillance (100) est configuré pour recevoir les données relatives à la délivrance de nicotine en provenance des au moins deux distributeurs (20, 40, 60, 80) et pour calculer une quantité totale de nicotine délivrée, et
e. au moins l'un des distributeurs (40) est prévu sous la forme d'un distributeur de timbres (40) destiné à délivrer des timbres (50), le distributeur de timbres (40) étant rempli d'une pluralité de timbres (50) qui possèdent chacun un réservoir pourvu de nicotine, lequel repose contre la peau lors de l'application du timbre sur la peau d'un utilisateur,
et/ou
au moins l'un des distributeurs (60) est réalisé sous la forme d'un distributeur de chewing-gums (60), le distributeur de chewing-gums (60) étant rempli d'une pluralité de chewing-gums (70) qui contiennent chacun de la nicotine.

2. Système (10) selon la revendication 1, avec la caractéristique supplémentaire suivante :
a. l'appareil de surveillance (100) possède un dispositif d'affichage (102) et il est configuré pour représenter la quantité totale de nicotine délivrée sur le dispositif d'affichage (102).

3. Système (10) selon l'une des revendications précédentes, avec la caractéristique supplémentaire suivante :
a. l'appareil de surveillance (100) est configuré pour calculer la quantité totale sur une période limitée, en particulier sur une journée.

4. Système (10) selon l'une des revendications précédentes, avec au moins l'une des caractéristiques supplémentaires suivantes :
a. l'appareil de surveillance (100) est configuré pour comparer la quantité totale avec une quantité maximale prédéfinie et pour délivrer un message d'avertissement lorsque la quantité totale est atteinte et/ou dépassée, et/ou
b. l'appareil de surveillance (100) est configuré pour comparer la quantité totale avec une quantité maximale prédéfinie et pour calculer la quantité de décharge qui est encore disponible le jour actuel, dans la semaine actuelle ou dans le mois actuel.

5. Système (10) selon l'une des revendications précédentes, avec la caractéristique supplémentaire suivante :
a. au moins l'un des distributeurs (20) est réalisé sous la forme d'un distributeur de liquide (20) destiné à délivrer de la nicotine sous forme liquide en tant que jet non atomisé, de jet pulvérisé ou sous forme de gouttelettes,
en particulier avec au moins l'une des caractéristiques supplémentaires suivantes :
b. l'au moins un distributeur de liquide (20) possède une poignée d'actionnement (22) et il est configuré pour détecter un actionnement de la poignée d'actionnement (22), et/ou
c. l'au moins un distributeur de liquide (20) possède un appareil rapporté (30), lequel est couplé de manière amovible à une partie boîtier (26) du distributeur de liquide (20) et est configuré pour détecter la manipulation du distributeur de liquide (20), et/ou
d. l'au moins un distributeur de liquide (20) possède un réservoir de liquide (24) dont le contenu comprend de la nicotine, et/ou
e. l'au moins un distributeur de liquide (20) est réalisé sous la forme d'un distributeur à pompe.

6. Système (10) selon l'une des revendications précédentes, avec au moins l'une des caractéristiques supplémentaires suivantes :
a. le distributeur de timbres (40) possède un espace de réception (42) destiné à recevoir les timbres (50), un élément de recouvrement mobile (44) étant prévu, qui, dans une position ouverte, permet d'accéder à l'espace de réception (42) et de prélever un timbre (50), le distributeur de timbres (40) possédant de préférence un capteur (46) servant à détecter la position ouverte, et/ou
b. le distributeur de timbres (40) possède une poignée d'actionnement (52) au moyen de laquelle un timbre individuel (50) peut être prélevé du distributeur de timbres (40), le distributeur de timbres (40) possédant de préférence un capteur (54) servant à détecter l'actionnement de la poignée d'actionnement (52).

7. Système (10) selon l'une des revendications précédentes, avec au moins l'une des caractéristiques supplémentaires suivantes :
a. au moins l'un des distributeurs (60) est réalisé sous la forme d'un distributeur de comprimés (60), et/ou
b. le distributeur de chewing-gums (60) ou le distributeur de comprimés (60) possède une coque (61) dans laquelle est inclus un moyen d'emballage (71) avec des chewing-gums (70) ou des comprimés (70) individuels, le moyen d'emballage (71) pouvant être tiré hors de la coque (61) pour le prélèvement d'un chewing-gum (70) ou d'un comprimé (70) et, de préférence, l'extraction du moyen d'emballage (71) ou la libération d'un dispositif de blocage afin de pouvoir extraire le moyen d'emballage (71) pouvant être détectée au moyen d'un capteur (66), et/ou
c. le distributeur de chewing-gums (60) ou le distributeur de comprimés (60) possède un espace de réception (62) destiné à recevoir les chewing-gums (70) ou les comprimés (70), un élément de recouvrement mobile (64) étant prévu, qui, dans une position ouverte, permet d'accéder à l'espace de réception (62) et de prélever un chewing-gum (70) ou un comprimé (70), le distributeur de chewing-gums (60) ou le distributeur de comprimés (60) possédant de préférence un capteur (66) servant à détecter la position ouverte, et/ou
d. le distributeur de chewing-gums (60) ou le distributeur de comprimés (60) possède une poignée d'actionnement (72) au moyen de laquelle un chewing-gum (70) individuel ou un comprimé (70) individuel peut être prélevé du distributeur de chewing-gums (60) ou du distributeur de comprimés (60), le distributeur de chewing-gum (60) ou le distributeur de comprimés (60) possédant de préférence un capteur (74) servant à détecter l'actionnement de la poignée d'actionnement (72).

8. Système (10) selon l'une des revendications précédentes, avec la caractéristique supplémentaire suivante :
a. au moins l'un des distributeurs (80) est configuré pour délivrer des cartouches de liquide (90), ces cartouches de liquide (90) étant prévues pour être couplées à un arrangement de décharge (95), de préférence avec au moins l'une des caractéristiques supplémentaires suivantes :
b. le distributeur (80) possède un espace de réception (82) destiné à recevoir les cartouches de liquide (90), un élément de recouvrement mobile (84) étant prévu, qui, dans une position ouverte, permet d'accéder à l'espace de réception (82) et de prélever une cartouche de liquide (90), le distributeur (80) possédant de préférence un capteur (86) servant à détecter la position ouverte, et/ou
c. le distributeur (80) possède une poignée d'actionnement (92) au moyen de laquelle une cartouche de liquide individuelle (90) peut être prélevée du distributeur (80), le distributeur (80) possédant de préférence un capteur (94) servant à détecter l'actionnement de la poignée d'actionnement (92).

9. Système (10) selon l'une des revendications précédentes, avec les caractéristiques supplémentaires suivantes :
a. au moins l'un des au moins deux distributeurs (20, 40, 60, 80) est prévu sous la forme d'un distributeur de timbres (40) et au moins l'un des au moins deux distributeurs est réalisé sous la forme d'un distributeur de liquide (20), et/ou
b. au moins l'un des au moins deux distributeurs (20, 40, 60, 80) est prévu sous la forme d'un distributeur de timbres (40) et au moins l'un des au moins deux distributeurs est réalisé sous la forme d'un distributeur de chewing-gums (60) ou d'un distributeur de comprimés (60), et/ou
c. au moins l'un des au moins deux distributeurs (20, 40, 60, 80) est prévu sous la forme d'un distributeur de timbres (40) et au moins l'un des au moins deux distributeurs est réalisé sous la forme d'un distributeur (80) destiné à délivrer des cartouches de liquide (90), et/ou
d. au moins l'un des au moins deux distributeurs (20, 40, 60, 80) est prévu sous la forme d'un distributeur de liquide (20) et au moins l'un des au moins deux distributeurs est réalisé sous la forme d'un distributeur de chewing-gums (60) ou d'un distributeur de comprimés (60), et/ou
e. au moins l'un des au moins deux distributeurs (20, 40, 60, 80) est prévu sous la forme d'un distributeur de liquide (20) et au moins l'un des au moins deux distributeurs est réalisé sous la forme d'un distributeur (80) destiné à délivrer des cartouches de liquide (90), et/ou
f. au moins l'un des au moins deux distributeurs (20, 40, 60, 80) est prévu sous la forme d'un distributeur de chewing-gums (60) ou d'un distributeur de comprimés (60) et au moins l'un des au moins deux distributeurs est réalisé sous la forme d'un distributeur (80) destiné à délivrer des cartouches de liquide (90).

10. Système (10) selon l'une des revendications précédentes, avec au moins l'une des caractéristiques supplémentaires suivantes :
a. la connexion de données est une connexion de données WLAN ou Bluetooth ou une connexion radio mobile GSM, 3G, 4G ou 5G, et/ou
b. l'appareil de surveillance (100) et au moins l'un des distributeurs (20, 40, 60, 80) sont configurés pour un processus de couplage au cours duquel un identificateur unique au niveau du distributeur est lu par le smartphone, et/ou
c. l'appareil de surveillance (100) est formé par un ordinateur, notamment un smartphone (100) ou une tablette électronique.

11. Procédé de surveillance de la consommation de nicotine depuis plusieurs distributeurs (20, 40, 60, 80) sous différentes formes d'application, ayant les caractéristiques suivantes :
a. au niveau d'au moins deux distributeurs (20, 40, 60, 80) qui sont configurés pour délivrer la nicotine sous différentes formes d'application, la manipulation respective du distributeur (20, 40, 60, 80) est détectée par l'intermédiaire de capteurs, et
b. en réaction à cela, les au moins deux distributeurs (20, 40, 60, 80) envoient des données relatives à la délivrance de la nicotine à un appareil de surveillance (100), et
c. l'appareil de surveillance (100) reçoit les données et calcule, sur la base de celles-ci, une quantité totale de la nicotine délivrée par les au moins deux distributeurs (20, 40, 60, 80), et
d. au moins l'un des distributeurs (40) est prévu sous la forme d'un distributeur de timbres (40) destiné à délivrer des timbres (50), le distributeur de timbres (40) étant rempli d'une pluralité de timbres (50) qui possèdent chacun un réservoir pourvu de nicotine, lequel repose contre la peau lors de l'application du timbre sur la peau d'un utilisateur,
et/ou
au moins l'un des distributeurs (60) est réalisé sous la forme d'un distributeur de chewing-gums (60), le distributeur de chewing-gums (60) étant rempli d'une pluralité de chewing-gums (70) qui contiennent chacun de la nicotine.

12. Procédé selon la revendication 11 avec les caractéristiques supplémentaires suivantes :
a. au moins l'un des distributeurs (20, 40, 60, 80) détecte une manipulation du distributeur, qui sert à ouvrir un espace de réception (42, 62, 62, 82) et, en réaction à cela, envoie des données à l'appareil de surveillance.

13. Procédé selon la revendication 11 ou 12, avec les caractéristiques supplémentaires suivantes :
a. au moins l'un des distributeurs (20, 40, 60, 80) détecte une manipulation du distributeur, qui provoque la décharge de nicotine et, en réaction à cela, envoie des données à l'appareil de surveillance,
en particulier avec au moins l'une des caractéristiques supplémentaires suivantes :
b. le distributeur détecte, au moyen d'un capteur, l'application d'une force et/ou le déplacement d'une poignée d'actionnement, et/ou
c. la décharge s'effectue sous forme liquide, notamment sous forme d'un jet non atomisé, d'un jet pulvérisé ou de gouttelettes, ou
d. la décharge s'effectue sous forme d'un timbre (50), d'un chewing-gum (70) ou d'un comprimé qui est pris ou appliqué par un utilisateur après la décharge, ou
e. la décharge s'effectue sous la forme d'une cartouche de liquide (90) qui est insérée dans un applicateur après la décharge.
